# EUROPEAN PATENT SPECIFICATION

(11) **EP 1 737 304 B1**
(45) Date of publication and mention of the grant of the patent: **07.05.2014**
(21) Application number: 05737679.0
(22) Date of filing: 08.04.2005
(51) Int. Cl.: A23L 1/29

(54) **LIQUID CONCENTRATED FORMULA**
KONZENTRIERTE FLÜSSIGFORMULIERUNG
FORMULE CONCENTREE LIQUIDE

(30) Priority: 09.04.2004 EP 04076132
(43) Date of publication of application: 03.01.2007
(73) Proprietor: N.V. Nutricia, 2712 HM Zoetermeer (NL)
(72) Inventor: MOLENAAR, Marike, Joanne, Bernadette, Warrington WA3 6DJ (GB)
(74) Representative: Nederlandsch Octrooibureau
(86) International application number: PCT/NL2005/000269
(87) International publication number: WO 2005/096845

(56) References cited:
- EP-A- 0 747 395
- EP-A- 0 756 827
- WO-A-02/15719
- WO-A-2004/026294
- US-A- 5 683 984
- US-B1- 6 200 950
- US-B1- 6 423 354
- DATABASE WPI Section Ch, Week 200327 Derwent Publications Ltd., London, GB; Class A92, AN 1996-405976 XP002294223 & JP 03 393946 B2 (TERUMO CORP) 7 April 2003 (2003-04-07)

## Description

The present invention is concerned with concentrated liquid nutritional products, process for the preparation thereof and methods for the treatment of cachexia, i.e. a state of serious constitutional disorder associated with malnutrition.

### Clinical problem

Some patients need complete nutrition in the smallest volume of liquid. These patients can be cachectic patients or persons suffering from end-stage AIDS, cancer or cancer treatment, severe pulmonary diseases like COPD (chronic obstructive pulmonary disease), tuberculosis and other infection diseases or persons that experienced severe surgery or trauma like burns. Furthermore, persons suffering from disorders in the throat or mouth such as oesophageal cancer or stomatitis and persons that have problems with swallowing like dysphagic persons, require special liquid, low-volume nutrition. Also, persons just suffering from reduced appetite or loss of taste will benefit from low-volume liquid food.

These groups of patients are extremely sensitive to food consistency and the organoleptic properties of the product like viscosity, mouth feel, taste, smell, colour, etc. In addition, these groups of patients may have become sensitive to lactose or gluten which may result in allergic reactions which can be badly tolerated.

The problem underlying the invention is to provide a liquid product which is attractive to the patient to consume, which has an extremely high nutrient density, and which supports nutrition and well-being in the patient groups mentioned above.

### Prior art

Many attempts have been made to solve this problem. Liquid ready-to-drink products are currently sold that comprise up to 28 wt.% dry matter. Inclusion of more ingredients leads to precipitates and sedimentation of ingredients, which imparts nutrient intake. Concentrating liquids also increases the chance of undesired interactions between ingredients which reduces stability, especially during heating and long-term storage. Increasing the amount of dry matter also increases viscosity. These phenomena often follow non-linear kinetics and the problems quickly increase in magnitude when the concentration of ingredients is increased above 28%.

Many of the commercial products that are currently available therefore have protein levels below 7.5 %. When one desires to increase the protein levels to a higher level, one replaces part of the total protein by peptides or free amino acids. This seriously decreases taste appreciation and therefore voluntary intake of the product by the patient group.

Many concentrates like condensed milks suffer from an incomplete nutrient profile, too high lactose levels, sticky mouth-feel, high viscosity, extreme sweetness and a high osmotic value, which is not appreciated by the consumer and increases rapidly feelings of fullness and satiety after consumption. This makes that the urge to consume more volume deteriorates rapidly once a small amount of the product has been consumed.

Cachectic patients typically suffer from extreme weakness, which often prevents sitting in a vertical position and impairs their ability to drink the food from a carton or even to suck it from a straw.

The manufacture of suitable concentrated complete nutrition is difficult. The ingredients to be used must fulfil many criteria: they must be pure and not be contaminated or spoiled. They must be accepted as food-grade, and not cause allergic reactions, especially against lactose or gluten. The amounts used should provide all nutrients to fulfil nutritional requirements, such as demands for specific amino acids, vitamins, minerals and fatty acids. Components that are deleterious to the patients such as oxidised products, or that may result in the creation of off-flavours must be avoided.

Dissolving of state of the art ingredients leads to one or more of the following problems either during shelf life or during processing, especially the heat treatment: precipitation, coagulation of protein, non-emulsifying properties, creaming, gelling or high viscosity. That is why in most prior art products the level of intact protein is typically well below 8%.

Inclusion of high amounts of soluble carbohydrates decreases the rate of dissolution of minerals, proteins and other soluble components and is therefore typically adjusted to below 19 g/100 ml. Fibres, especially soluble ones, further decrease dissolution rate and may increase viscosity further.

The energy density of complete liquid formulae is typically below 1.4 kcal/ml, which in most cases is a practical solution to fulfil nutritional and functional requirements. When the energy density must be higher (higher than 1.45 kcal/mole), one may use additional amounts of lipids. However, increasing the amount of lipids gives problems with homogeneity and/or thick consistencies.

EP 0 747 395 describes a product for treating renal patients having an energy density of 1.6-2.25 kcal/ml and comprising free amino acids and whey protein, wherein the ratio of essential to non-essential amino acids is 2-4 : 1. By using free amino acids the amino acid composition is improved without increasing the viscosity. However the taste is not acceptable for cachectic persons or other persons which have difficulties in eating the proper volume of food. The amount of protein is about 3-4 g/100 ml product.

WO 2004/026294 discloses a nutritional composition for promoting muscle protein synthesis, which may have a caloric density of about 1.5 kcal/ml, and intact protein may be casein at a level of 7.3 or 7.6 g per 100 ml. The remainder of the protein content is provided by free amino acids.

US 5,683,984 discloses nutritional compositions suitable for tube feeding, wherein native micellar casein as the protein. The micellar casein can be prepared by microfiltering skimmed milk.

WO 99/42001 (US 6,200,950) discloses a nutritional product which has an energy density of 1.4-1.8 kcal/ml, a carbohydrate content (maltodextrins) of 19 g, a protein content of 6 g and a fat content of 5.9 g per 100 ml. The protein is hydrolysed whey protein, which gives high amounts of short peptides and amino acids. Therefore, this product does not meet the requirements for patients only capable of ingesting small volumes of highly palatable nutrition.

Other concentrated liquid nutritional products of the prior art contain soy protein as protein source and corn syrup as carbohydrate source in order to reach sufficient density. However, soy protein does not provide an optimum amino acid profile and has a poor taste, and corn syrup gives excessive sweetness, due to the high fructose level. Therefore, such products can only be administered as an occasional energy support rather than as a complete food.

The problem to be solved according to the invention is to provide a nutritional composition suitable for feeding persons having ingestion problems, such as cachetic patients, having the following characteristics:
- a balanced composition allowing it to be consumed as the only food source;
- a liquid composition with relatively low viscosity, allowing easy intake;
- a high density of at least 1.45 kcal/ml, such that less than 1 litre is sufficient for daily food requirement;
- a sufficient level of essential amino acids, especially lysine, methionine and cysteine;
- an acceptable taste, with minimum presence of free amino acids and short peptides;
- a relatively low osmolarity, so as to allow quick passage through the stomach;
- a very low lactose level, in order to prevent allergic problems and discolouring problems.

### Description of the invention

It has now been found that a thin liquid enteral nutritional product can be produced that has desirable organoleptic properties, has a very high nutrient density, is effective and is produced without problems. The nutritional composition of the invention is defined in the appended claims.

The product of the invention is a liquid composition, which contains in g per 100 ml product:

| *Component* | *General* | *Preferred* | *Most preferred* | *Example* |
|---|---|---|---|---|
| Dry matter | 30-39 | 31-39 | 33-38 | 36.7 |
| Intact protein | 8.2-11 | 8.2-11 | 8.5-10 | 9.0 |
| Lipids | 4.5-8.0 | 4.8-7.0 | 5.0-6.0 | 5.3 |
| Carbohydrate | 17-27 | 18-25 | 19-23 | 21 |
| RDA vitamins, minerals, trace elements | | | | |

The product is preferably nutritionally complete, which means that all vitamins are included in a recommended dosage of at most 1500 ml per day of the composition. Also, vitamins (e.g. including folic acid, vitamins B6 and B12 and panthotenic acid) can be incorporated in an amount between 0.6 and 1.5 times the amount given in the example below. The invention also pertains to a dry (powder) or concentrated product, which, after reconstitution with water, results in the thin liquid product described above.

In one aspect, the invention provides a balanced, liquid concentrated food product having an improved protein composition. In another, independent aspect, the invention provides a balanced, liquid concentrated food product having an improved carbohydrate composition.

### Protein fraction:

The protein fraction provides 8.5-12 g per 100 ml, corresponding to about 0.31-0.48 kcal/ml (1.30-1.59 kJ/ml). The amounts of essential amino acids fulfils nutritional requirements: typically the amount of essential amino acids (Val, Leu, Ile, Met, Phe, Trp, Thr, His, Lys) to non-essential amino acids (Gly, Ala, Pro, Tyr, Ser, Cys, Asn, Asp, Gln, Glu, Arg) will be in the range of 0.6-1.1, preferably 0.75-1.05, most preferably 0.85-1.0 (w/w). Furthermore, the protein fraction preferably provides at least 8.6 wt.% of lysine residues, at least 2.5 wt.% of methionine residues and at least 0.5 wt.% of cysteine residues, more preferably at least 10 wt.% Lys, at least 2.7 wt.% Met and/or at least 1.0 wt.% Cys.

The protein fraction preferably contributes 19 to 33 % of the energy content of the composition, preferably 20 to 27 energy %. The protein content is largely (typically at least 75%, preferably at least 90%) composed of intact protein only. Preferably, the amount of free amino acids is below 5 wt.%, especially below 2 wt.% of the total protein content, and preferably below 0.4 g, especially below 0.2 g per 100 ml product. Preferably, the protein is from dairy origin for at least 90 wt.%. Casein can be a major protein component, e.g. accounting for 60-90 % of the protein. On the other hand, 10-40%, preferably 20-37 wt.%, or even 25-37 wt.%, most preferably 25-35 wt.% is whey protein.

The major part of the proteins is obtained by demineralisation of defatted (skimmed) milk. This major part is at least 70 wt.%, preferably at least 80 wt% of the protein fraction. It was found that such a protein fraction allows high concentrations in a complete food concentrate without excessive viscosity problems, while combinations of caseinate products and whey protein preparations result in high viscosities. The demineralisation is preferably performed in such a manner that at least 97%, especially at least 99% of lactose is removed from the milk, leaving essentially intact protein; the protein is enriched in calcium relative to sodium, potassium and other electrolytes. This can be done using extended ultrafiltration. Such demineralised milk protein are sometimes referred to as milk protein isolates.

The protein fraction of the composition of the invention can also be obtained by demineralising casein in a similar way, and then optionally adding whey proteins (whey protein isolate). Other proteins, such as vegetable proteins like proteins from lupine, maize, rice, soy, pea or potato, can also be present, although it is preferred that these do not account for more than 10% (w/w), especially not more than 5%, of the protein content of the composition.

### Lipid fraction:

The lipid fraction provides from about 41 kcal up to about 72 kcal (4.5-8.0 g) per 100 ml. It preferably accounts for 23-40% energy of the energy content of the composition, preferably from 28 to 35 en%. It is preferred that the lipid fraction contains 30-60 wt.% animal or algal fat, 40-70 wt.% vegetable fat and optionally 0-20 wt% medium-chain triglycerides (mainly C8 and C10 triglycerides; MCT). The animal fat fraction should preferably be low in milk fat, i.e. the milk fat content of the fat fraction is below 6%, especially below 3%. In particular a mixture of corn oil, egg oil and/or canola oil and specific qualities of marine oils can be used. Egg oils, fish oils and algal oils are the preferred source of non-vegetable fats. In order to prevent formation of off-flavours and to decrease a fishy after-taste, it is recommended to select ingredients that are relatively low in docosahexaenoic acid (DHA), i.e. less than 6, preferably less than 4 wt% of DHA with respect to the lipid fraction. Marine oils containing DHA are preferably present in the product at a level of below 25% of the lipid fraction, more preferably below 15%. On the other hand, inclusion of eicosapentaenoic acid (EPA) is highly desirable for obtaining the maximum health effect. The EPA weight proportion of the lipid fraction is preferably between 4 and 15 %, more preferably between 8 and 13 %, leading to a daily dosage of 0.8-2.3 g, most preferably 1.2-1.9 g, when administered in a volume of 375 ml food composition. The weight ratio between EPA and DHA is advantageously at least 6:4, for example between 2:1 and 10:1.

### Carbohydrate fraction:

The carbohydrate fraction amounts to 17-27 g per 100 ml product (2.84-4.51 kJ/ml). The carbohydrate fraction comprises digestible and preferably also non-digestible carbohydrates. The digestible carbohydrate fractions provide about 17-24 g per 100 ml (0.68-1.0 kcal/ml; 2.84-4.01 kJ/ml), preferably 17.5-23.5 and especially 18-22 g/100 ml. The carbohydrate fraction preferably accounts for 38-66% of the energy content of the composition, more preferably from 40 to 60 en%, especially 45-55 en%.

The composition of the carbohydrate fraction should be such that high viscosities, excessive sweetness, excessive browning (Maillard reactions) and excessive osmolarities are avoided. Acceptable viscosities and osmolarities are achieved by adjusting the average chain length (average degree of the polymerization, DP) of the carbohydrates between 1.5 and 6, preferably between 1.8 and 4. In order to avoid excessive sweetness, the total level of sucrose and fructose is less than 52% and preferably less than 40% of the carbohydrate fraction, especially of the digestible carbohydrate fraction. Long-chain digestible carbohydrates such as starch, starch fractions and mild starch hydrolysates (DP ≥ 6, DE < 20)), may also be present, but preferably in an amount of less than 25 wt.%, especially less than 15 wt.% of the carbohydrate fraction, and less than 6 g, preferably less than 4 g per 100 ml liquid food product.

Furthermore, it is preferred that the reducing power of the total carbohydrate fraction is less than 40%, especially less than 30%, most preferably 25% or less, of the reducing power of an equivalent amount by weight of glucose. The combined requirements of low viscosity and low reducing power implies that the carbohydrate fraction contains between 20 and 75 wt.%, preferably between 25 and 65 wt.% of non-reducing digestible mono-, di- and trisaccharides, especially mono- or disaccharides, most preferably disaccharides. Suitable examples of such non-reducing saccharides are sucrose (although its level is preferably limited to below 35% to avoid excessive sweetness), and trehalose. The preferred amount of trehalose, optionally together with other non-reducing sugars, but excluding sucrose, is preferably 12-50, more preferably 18-45 wt.% of the carbohydrate fraction, more in particular 13-55, especially 20-50 wt.% of the digestible carbohydrate fraction. In particular a mixture of glucose syrups, trehalose and sucrose can be used.

In addition to the energy-providing carbohydrates mentioned above, the carbohydrate fraction preferably also contains non-digestible carbohydrates (dietary fibres), in an amount of 0.5-6 g per 100 ml of the composition. The fibres include non-digestible saccharides having an average DP of 2-20, preferably 2-10. More preferably, these oligosaccharides do not contain substantial amounts (less than 5 wt.%) of saccharides outside these DP ranges, and are soluble. These can include e.g. fructo-oligosaccharides (FOS), trans-galacto-oligosaccharides (TOS), xylo-oligosaccharides (XOS), soy oligosaccharides, etc. Optionally also higher molar weight compounds such as inulin, cellulose, resistant starch and the like can be incorporated. The amount of insoluble fibre such as cellulose is preferably below 20 wt% of the fibre component and/or below 0.4 g per 100 ml. The amount of thickening polysaccharides such as carrageenans, xanthans, pectins, galactomannans and other high molecular weight (DP > 50) indigestible polysaccharides is preferably low, i.e. less than 20% of the fibre composition or less than 1 g per 100 ml. Instead, hydrolysed polysaccharides such as hydrolysed pectins and galactomannans can be advantageously included.

A preferred fibre component is indigestible oligosaccharides having a chain length (DP) of 2 to 10, for example Fibersol (resistant oligoglucose), in particular hydrogenated Fibersol, or a mixture of oligosaccharides having a chain length of 2-10, such as fructo-oligosaccharides or galacto-oligosaccharides, which may also contain a small amount of higher saccharides (e.g. DP 11-20). Such oligosaccharides preferably comprise 50-90 wt. of the fibre fraction or 0.5-3 g per 100 1 of the composition. Other suitable fibre components include saccharides that have only partial digestibility or partial reducing power, for example ketose-terminated oligosaccharides such as tagatose, palatinose and trehalulose, oligodextroses of the *Litesse Ultra*® type, and the like.

The following carbohydrate composition is especially preferred:

| Carbohydrate component | wt.% of carbohydrate fraction | | wt. (g) per 100 ml of food composition | |
|---|---|---|---|---|
| | Broad | preferred | broad | preferred |
| maltodextrins (DP 2-6, DE 20-60) | 30-65 | 35-60 | 5-14 | 7-12 |
| sucrose | 5-35 | 12-30 | 1-8 | 2.5-7 |
| other non-reducing mono/di/trisaccharides, especially trehalose | 15-45 | 20-40 | 3-9 | 4-8 |
| lactose | 0-1 | 0-0.5 | 0-0.2 | 0-0.1 |
| reducing monosaccharides (e.g. glucose, fructose) | 0-10 | 0-5 | 0-2.5 | 0-1.5 |
| fibres | 0-20 | 0-12 | 0-6 | 0-4 |

Describing the carbohydrate fraction as a function of sugar size, it comprises 0-15 wt.% (pref. 2-10 wt.%) of monosaccharides (e.g. glucose), 50-100 wt.% (pref. 60-90 wt.%) of disaccharides (e.g. maltose, sucrose, trehalose), 0-40 wt.% (pref. 10-25 wt.%) of trisaccharides (e.g. maltotriose, kestose, raffinose), 0-30 wt-% (pref. 5-20 wt.%) of DP 4-6 oligosaccharides and 0-25 wt.% (pref. 0-15 wt.%) of higher saccharides.

### Further components

The amount of divalent ions is 170-230 mg per 100 ml and preferably 180-220 mg. Preferably the amount of calcium is between 155 and 185 mg/100 ml and preferably between 160 and 180 mg. The phosphorus content can be above 10 mg per g protein, with a calcium to phosphorus weight ratio between 1.0 and 2.0, preferably between 1.1 and 1.7. Carnitine may advantageously be present in an amount of 8-1000 mg per 100 ml, preferably 10-100 mg per 100 ml product; it may have the form of carnitine, alkyl carnitines, acyl carnitines or other carnitine derivatives- Organic acids are preferably present at a level of between 0.1 and 0.6 g per 100 ml, especially 0.25-0.5 g/100ml. These acids include short fatty acids such as acetic acid, hydroxy acids such as lactic acid, gluconic acid, and preferably polyvalent hydroxy acids such as malic acid and citric acid.

The viscosity of the liquid composition should be low, i.e. below 50 mPa.s at 20°C at a shear rate of 100 s⁻¹, preferably between 20 and 40 mPa.s. The osmolarity of the composition is preferably between 360 and 480 mOsm, preferably between 390 and 430 mOsm. The density of the composition will be between 1.05 and 1.18 g/ml, especially between 1.08 and 1.15 g/ml. The energy density of the liquid composition is at least 1.45 kcal/ml, up to about 2.25 kcal/ml (6.06 - 9.4 kJ/ml), more preferably at least 1.52, up to less than 2.0 kcal/ml (6.35 - 8.35 kJ/ml).

The product of the invention is a thin liquid and it is preferred that it is packaged in small individual dosage units. Preferred package units are 5-250 ml, especially 80-200 ml, in particular 100-150 ml, or alternatively, 5-30 ml. These packaging sizes for liquid food products containing at least 1.45 kcal per ml, and containing at least 8.5 g protein per 100 ml, were found to be very effective in administering sufficient food to patients having food ingestion problems, and constitute a special embodiment of the invention. The liquid should contain 8.5 g or more protein per 100 ml, and preferably contains one or more of the components described above, such as specific carbohydrates, specific fats, vitamins and the like. The packaging can e.g. have the form of a block-shaped carton to be emptied with a straw, a carton or plastic beaker with removable cover, or a small-sized bottle for the 80-200 ml range, and e.g. small cups for the 10-30 ml range. Another suitable packaging mode is inclusion of small volumes of liquid (e.g. 10-20 ml) in edible solid or semi-solid hulls or capsules, for example chocolate coverings (bonbon type), gelatine-like coverings etc.

### Preparation of the product

The product can be prepared for example by first preparing a liquid protein fraction. Thus, a milk ultrafiltration retentate in dry form can be dissolved in an aqueous solution to obtain a low-viscosity liquid. Then the carbohydrates (e.g. maltodextrins, sucrose, trehalose, and optionally indigestible oligosaccharides), and water-soluble vitamins and other components are added in one or two stages, mixed, adjusted to the desired viscosity, and then the fat fraction, including fat-soluble vitamins, is added, homogenised, heat-treated and packaged.

### Effectivity

The compositions according to the invention are suitable for any patients having problems in ingesting substantial amounts of food, such as cachexia patients. The administration of these composition lead to a higher food intake, resulting in less loss of lean body mass, improvements in well-being and less complications during the diseased state. Compliance of the product of the invention, i.e. the proportion of supplied energy to consumed energy, is improved with respect to conventional products.

### Example

Product composition intended for cachectic patients:

| *Component* | *Amount per 10O ml product* | | |
|---|---|---|---|
| Energy | 160 | | |
| Protein (g) | 9,0 | | |
| Casein (g) | 6,1 | | |
| Whey protein (g) | 2,9 | | |
| Fat (g) | 5,3 | | |
| EPA (g) | 0,67 | | |
| n-6/n-3 ratio (w/w) 1,14 | | | |
| Carbohydrate (g) | 22,1 | | |
| maltodextrins DE 39 | 4,6 | | |
| maltodextrins DE 47 | 4,6 | | |
| sucrose | 4,4 | | |
| trehalose | 4,5 | | |
| soluble fibre | 1,7 | | |
| insoluble fibre | 0,3 | | |
| Na (mg) | | 110 | |
| K (mg) | | 215 | |
| Ca (mg) | | 170 | |
| Mg (mg) | | 28,2 | |
| Cl (mg) | | 140 | |
| P (mg) | | 120 | |
| Fe (mg) | | 1,9 | |
| Zn (mg) | | 2,05 | |
| Cu (µg) | | | 288 |
| Mn (µg) | | | 680 |
| F (µg) | | | 160 |
| Mo (µg) | | | 16 |
| Se (µg) | | | 13,5 |
| Cr (µg) | | | 11 |
| I (µg) | | | 21 |
| Carnitine (mg) | | 11 | |
| Choline (mg) | | 59 | |
| Taurine (mg) | | 13 | |
| Niacine (mg NE) | | 2,9 | |
| Vitamin C (mg) | | 30 | |
| Vitamin E (mg TE) | | 3,0 | |
| Carotenoids (µg) | | | 320 |
| Vitamin A (µg RE) | | | 130 |
| Vitamin D (µg) | | | 1,1 |
| Vitamin K (µg) | | | 8,5 |
| Vitamin B1 (µg) | | | 240 |
| Vitamin B2 (µg) | | | 250 |
| Vitamin B6 (µg) | | | 680 |
| Vitamin B12 (µg) | | | 0,95 |
| Folic acid (µg) | | | 66 |
| Biotin (µg) | | | 6,4 |
| Panthotenic acid (µg) | | | 850 |
| Carotenoids (µg) | | | 320 |

## Claims

1. A liquid complete nutritional composition suitable for feeding cachectic patients, having an energy density of at least 1.45 kcal/ml (at least 6.06 kJ/ml), comprising:
- a carbohydrate fraction in an amount of 17-27 g per 100 ml;
- a protein fraction in an amount of 8.5-12 g per 100 ml, which comprises intact protein in an amount of 8.2-11 g per 100 ml; and
- a lipid fraction;
***characterised* in that** at least 70 wt.% of the protein fraction is obtained by demineralising milk, and the protein fraction comprises between 25 and 37 wt.% of whey proteins.

2. A liquid composition according to claim 1, in which said demineralising is achieved by ultrafiltration.

3. A liquid composition according to claim 1 or 2, ***characterised* in that** the carbohydrate fraction comprises:
- 0-35 wt.% of sucrose;
- 15-45 wt.% of other non-reducing mono-, di- and/or trisaccharides;
- 5-50 wt.% of other mono- and disaccharides;
- 5-40 wt.% of other trisaccharides and higher saccharides.

4. The composition according to claim 3, wherein the non-reducing disaccharides comprise trehalose.

5. A liquid complete nutritional composition suitable for feeding cachectic patients, having an energy density of at least 1.45 kcal/ml (at least 6.06 kJ/ml), comprising:
- a carbohydrate fraction in an amount of 17-27 g per 100 ml;
- a protein fraction in an amount of 8.5-12 g per 100 ml, which comprises intact protein in an amount of 8.2-11 g per 100 ml; and
- a lipid fraction;
***characterised* in that** at least 70 wt.% of the protein fraction is obtained by demineralising milk, and the protein fraction comprises less than 5 wt.% of free amino acids.

6. A liquid composition according to claim 5, wherein the protein fraction essentially consists of intact proteins and comprises 60-90 wt.% of caseins.

7. A liquid composition according to any one of claims 1-6, which comprises 0.5-6 g fibre per 100 ml.

8. A liquid composition according to any one of claims 1-7, wherein the protein fraction amounts to above 8.7 g per 100 ml.

9. A liquid composition according to any one of claims 1-8, in which the protein fraction contains at least 1.0 wt.% of cysteine residues.

10. A liquid composition according to any one of claims 1-9, in which the protein fraction comprises at least 8.6 wt.% of lysine residues, at least 2.5 wt.% of methionine residues and at least 0.5 wt.% of cysteine residues.

11. A liquid composition according to any one of claims 1-10, wherein the lipid fraction amounts to 5.0-7.0 g per 100 ml.

12. A liquid composition according to any one of claims 1-11, having a viscosity of the liquid of below 50 mPa.s at a shear rate of 100 s⁻¹ and a temperature of 20°C.

13. A liquid composition according to any one of claims 1-12, wherein the amount of digestible carbohydrates is 18-23.5 and preferably 18-22 g per 100 ml.

14. A packaged food product containing between 5 and 250 ml of the composition according to any of claims 1-13 in a unit package.

15. A process for preparing a liquid product according to any one of claims 1-13, comprising preparing a liquid protein fraction and subsequently mixing with a carbohydrate fraction and a fat fraction, **characterised by** dissolving in an aqueous solution a dry demineralised milk product, optionally together with a part of other water-soluble components, adjusting the suspension obtained to a viscosity value of below 50 mPa.s (at 100 s⁻¹) and then mixing an amount of this suspension with water or remaining ingredient, including the fat fraction, to arrive at the final composition.

## Patentansprüche

1. Flüssige Vollnährzusammensetzung, die zur Ernährung kachektischer Patienten geeignet ist und eine Energiedichte von wenigstens 1,45 kcal/ml (wenigstens 6,06 kJ/ml) aufweist, umfassend:
- eine Kohlenhydratfraktion in einer Menge von 17-27 g pro 100 ml;
- eine Proteinfraktion in einer Menge von 8,5-12 g pro 100 ml, die intaktes Protein in einer Menge von 8,2-11 g pro 100 ml umfasst; und
- eine Lipidfraktion;
**dadurch gekennzeichnet, dass** wenigstens 70 Gew.-% der Proteinfraktion durch Demineralisieren von Milch erhalten werden und die Proteinfraktion zwischen 25 und 37 Gew.-% Molkenproteine umfasst.

2. Flüssige Zusammensetzung gemäß Anspruch 1, wobei die Demineralisierung durch Ultrafiltration erreicht wird.

3. Flüssige Zusammensetzung gemäß Anspruch 1 oder 2, **dadurch gekennzeichnet, dass** die Kohlenhydratfraktion Folgendes umfasst:
- 0 bis 35 Gew.-% Saccharose;
- 15 bis 45 Gew.-% anderer nichtreduzierender Mono-, Di- und/oder Trisaccharide;
- 5 bis 50 Gew.-% anderer Mono- und Disaccharide;
- 5 bis 40 Gew.-% anderer Trisaccharide und höherer Saccharide.

4. Zusammensetzung gemäß Anspruch 3, wobei die nichtreduzierenden Disaccharide Trehalose umfassen.

5. Flüssige Vollnährzusammensetzung, die zur Ernährung kachektischer Patienten geeignet ist und eine Energiedichte von wenigstens 1,45 kcal/ml (wenigstens 6,06 kJ/ml) aufweist, umfassend:
- eine Kohlenhydratfraktion in einer Menge von 17-27 g pro 100 ml;
- eine Proteinfraktion in einer Menge von 8,5-12 g pro 100 ml, die intaktes Protein in einer Menge von 8,2-11 g pro 100 ml umfasst; und
- eine Lipidfraktion;
**dadurch gekennzeichnet, dass** wenigstens 70 Gew.-% der Proteinfraktion durch Demineralisieren von Milch erhalten werden und die Proteinfraktion weniger als 5 Gew.-% freie Aminosäuren umfasst.

6. Flüssige Zusammensetzung gemäß Anspruch 5, wobei die Proteinfraktion im Wesentlichen aus intakten Proteinen besteht und 60-90 Gew.-% Caseine umfasst.

7. Flüssige Zusammensetzung gemäß einem der Ansprüche 1 bis 6, die 0,5 bis 6 g Ballaststoffe pro 100 ml umfasst.

8. Flüssige Zusammensetzung gemäß einem der Ansprüche 1 bis 7, wobei die Proteinfraktion über 8,7 g pro 100 ml ausmacht.

9. Flüssige Zusammensetzung gemäß einem der Ansprüche 1 bis 8, wobei die Proteinfraktion wenigstens 1,0 Gew.-% Cysteinreste enthält.

10. Flüssige Zusammensetzung gemäß einem der Ansprüche 1 bis 9, wobei die Proteinfraktion wenigstens 8,6 Gew.-% Lysinreste, wenigstens 2,5 Gew.-% Methioninreste und wenigstens 0,5 Gew.-% Cysteinreste umfasst.

11. Flüssige Zusammensetzung gemäß einem der Ansprüche 1 bis 10, wobei die Lipidfraktion 5,0 bis 7,0 g pro 100 ml ausmacht.

12. Flüssige Zusammensetzung gemäß einem der Ansprüche 1 bis 11, die eine Viskosität der Flüssigkeit von unter 50 mPa· s bei einer Schergeschwindigkeit von 100 s⁻¹ und einer Temperatur von 20 °C aufweist.

13. Flüssige Zusammensetzung gemäß einem der Ansprüche 1 bis 12, wobei die Menge an verdaulichen Kohlenhydraten 18 bis 23,5 und vorzugsweise 18 bis 22 g pro 100 ml beträgt.

14. Verpacktes Lebensmittel, das zwischen 5 und 250 ml der Zusammensetzung gemäß einem der Ansprüche 1 bis 13 in einer Packungseinheit enthält.

15. Verfahren zur Herstellung eines flüssigen Produkts gemäß einem der Ansprüche 1 bis 13, umfassend das Herstellen einer flüssigen Proteinfraktion und das anschließende Mischen derselben mit einer Kohlenhydratfraktion und einer Fettfraktion, **dadurch gekennzeichnet, dass** man ein trockenes demineralisiertes Milchprodukt, gegebenenfalls zusammen mit einem Teil anderer wasserlöslicher Komponenten, in einer wässrigen Lösung auflöst, die erhaltene Suspension auf einen Viskositätswert von unter 50 mPa· s (bei 100 s⁻¹) einstellt und dann eine Menge dieser Suspension mit Wasser oder den restlichen Bestandteilen einschließlich der Fettfraktion mischt, um zu der endgültigen Zusammensetzung zu gelangen.

## Revendications

1. Composition nutritive liquide complète appropriée pour alimenter des patients cachectiques, ayant une densité énergétique d'au moins 1,45 kcal/ml (au moins 6,06 kJ/ml), comprenant :
- une fraction glucidique en une quantité de 17-27 g pour 100 ml ;
- une fraction protéique en une quantité de 8,5-12 g pour 100 ml, qui comprend une protéine intacte en une quantité de 8,2-11 g pour 100 ml ; et
- une fraction lipidique ;
***caractérisée* en ce qu'**au moins 70 % en poids de la fraction protéique est obtenue par une déminéralisation du lait, et la fraction protéique comprend entre 25 et 37 % en poids de protéines lactosériques.

2. Composition liquide selon la revendication 1, dans laquelle ladite déminéralisation est obtenue par ultrafiltration.

3. Composition liquide selon la revendication 1 ou 2, ***caractérisée* en ce que** la fraction glucidique comprend :
- 0-35 % en poids de saccharose ;
- 15-45 % en poids d'autres mono-, di- et/ou trisaccharides non réducteurs ;
- 5-50 % en poids d'autres mono- et disaccharides ;
- 5-40 % en poids d'autres trisaccharides et saccharides supérieurs.

4. Composition selon la revendication 3, dans laquelle les disaccharides non réducteurs comprennent le tréhalose.

5. Composition nutritive liquide complète appropriée pour alimenter des patients cachectiques, ayant une densité énergétique d'au moins 1,45 kcal/ml (au moins 6,06 kJ/ml), comprenant :
- une fraction glucidique en une quantité de 17-27 g pour 100 ml ;
- une fraction protéique en une quantité de 8,5-12 g pour 100 ml, qui comprend une protéine intacte en une quantité de 8,2-11 g pour 100 ml ; et
- une fraction lipidique ;
***caractérisée* en ce qu'**au moins 70 % en poids de la fraction protéique est obtenue par une déminéralisation du lait, et la fraction protéique comprend moins de 5 % en poids d'acides aminés libres.

6. Composition liquide selon la revendication 5, dans laquelle la fraction protéique consiste essentiellement en des protéines intactes et comprend 60-90 % en poids de caséines.

7. Composition liquide selon l'une quelconque des revendications 1-6, qui comprend 0,5-6 g de fibre pour 100 ml.

8. Composition liquide selon l'une quelconque des revendications 1-7, dans laquelle la fraction protéique est présente en une quantité supérieure à 8,7 g pour 100 ml.

9. Composition liquide selon l'une quelconque des revendications 1-8, dans laquelle la fraction protéique contient au moins 1,0 % en poids de résidus cystéine.

10. Composition liquide selon l'une quelconque des revendications 1-9, dans laquelle la fraction protéique comprend au moins 8,6 % en poids de résidus lysine, au moins 2,5 % en poids de résidus méthionine et au moins 0,5 % en poids de résidus cystéine.

11. Composition liquide selon l'une quelconque des revendications 1-10, dans laquelle la fraction lipidique est présente en une quantité de 5,0-7,0 g pour 100 ml.

12. Composition liquide selon l'une quelconque des revendications 1-11, ayant une viscosité du liquide inférieure à 50 mPa.s à une vitesse de cisaillement de 100 s⁻¹ et à une température de 20 °C.

13. Composition liquide selon l'une quelconque des revendications 1-12, dans laquelle la quantité de glucides digestibles est de 18-23,5 et de préférence de 18-22 g pour 100 ml.

14. Produit alimentaire conditionné contenant entre 5 et 250 ml de la composition selon l'une quelconque des revendications 1-13, dans un conditionnement unitaire.

15. Procédé destiné à préparer un produit liquide selon l'une quelconque des revendications 1-13, comprenant la préparation d'une fraction protéique liquide puis son mélange avec une fraction glucidique et une fraction de matières grasses, **caractérisé par** une dissolution, dans une solution aqueuse, d'un produit laitier déminéralisé sec, facultativement avec une partie d'autres composants solubles dans l'eau, un ajustement de la suspension obtenue à une valeur de viscosité inférieure à 50 mPa.s (à 100 s⁻¹) puis un mélange d'une quantité de cette suspension avec de l'eau ou un ingrédient restant, comprenant la fraction de matières grasses, afin d'obtenir la composition finale.
